# EUROPEAN PATENT APPLICATION

(11) **EP 1 967 184 A1**
(43) Date of publication of application: **10.09.2008**
(21) Application number: 06843648.4
(22) Date of filing: 28.12.2006
(51) Int. Cl.: A61K 9/26, A61K 9/14, A61K 31/4439, A61K 47/02, A61K 47/32, A61K 47/38, A61P 1/04, A61P 43/00

(54) **CONTROLLED RELEASE SOLID PREPARATION**

(30) Priority: 28.12.2005 JP 2005378061
(71) Applicant: Takeda Pharmaceutical Company Limited, Osaka-shi, Osaka 541-0045 (JP)
(72) Inventor: BANDO, Hiroto, Osaka-shi Osaka 541-0045 (JP); KURASAWA, Takashi, Osaka-shi Osaka 532-8686 (JP)
(74) Representative: Wright, Robert Gordon McRae
(86) International application number: PCT/JP2006/326270
(87) International publication number: WO 2007/074910

(57) **Abstract**

The present invention provides a controlled release solid preparation superior in then stability of an active ingredient, which can exhibit pharmacological effects steadily and rapidly after administration, and shows a sustained pharmacological effect for a prolonged period of time: a controlled release solid preparation containing a combination of (1) an antacid, (2) an immediate-release part containing a compound unstable to acid and a basic substance, and (3) a sustained-release part containing a compound unstable to acid and a basic substance, and having a film that dissolves at pH 6.5 or above.

## Description

### Technical Field

The present invention relates to a solid preparation. More particularly, the present invention relates to a controlled release solid preparation which is excellent in the stability of an active ingredient, can exhibit pharmacological effects steadily and rapidly after administration, and shows a sustained pharmacological effect for a prolonged period of time.

### Background Art

Since proton pump inhibitors (hereinafter sometimes referred to as PPI) such as benzimidazole compounds (e.g., lansoprazole; omeprazole, rabeprazole, pantoprazole, ilaprazole and the like) have a gastric acid secretion-inhibitory action, a gastric mucosa-protective action and the like, they have been widely used as therapeutic agents for peptic ulcer.
However, these compounds have poor stability, and are unstable to humidity, temperature, light, acid and the like. These compounds are particularly unstable to acid, and become extremely unstable as the pH of an aqueous solution or suspension thereof becomes low. When orally administered, therefore, these compounds may not be able to exhibit sufficient activity since they are decomposed by gastric acid and the like.
The stability of these compounds in preparations such as tablet, powder, fine granules, capsule and the like may become lower than that of the compounds themselves, due to strong interactions with other ingredient components in the preparation. As a result, color change and decomposition may be observed during production and preservation.
Various attempts have been made in the pharmaceutical preparations containing these compounds as active ingredients, so as to overcome the unstability of the compounds. For example, a tablet, granules or fine granules containing core particles containing PPI or a salt thereof or an optically active form thereof as an active ingredient, and a particular, pH-dependently soluble, release-controlling film (enteric film) have been disclosed (patent reference 1). Due to the enteric film, these preparations can suppress decomposition of the active ingredient by gastric acid and the like. However, since some time is required for dissolution of the enteric film in the gastrointestinal tract and absorption of the drug, rapid expression of the pharmacological effect in the early stage of the administration is hardly expected.
In the meantime, a gastrically-disintegrating solid preparation free of an enteric film, which contains an active ingredient unstable to acid and at least one kind of component selected from metal oxides and metal hydroxides is disclosed (patent reference 2). In addition, a chewable tablet free of an enteric film, which contains an active ingredient unstable to acid and at least one kind of component selected from alkaline earth metal carbonates, metal oxides and metal hydroxides is disclosed (patent reference 3). These preparations can suppress decomposition of the active ingredient by gastric acid and the like after administration, and are suitable for rapid expression of pharmacological effects upon administration. However, retention of the pharmacological effect for a prolonged period of time is difficult for these preparations.
Also, use of a basic inorganic salt for stabilization of the active ingredient in the preparation is disclosed (patent references 4 - 6, non-patent reference 1).
Furthermore, various preparations having plural different release control systems in combination are disclosed for the rapid expression of the pharmacological effect after administration and retention of the pharmacological effect for a prolonged period of time (patent references 7 - 11).
patent reference 1: JP-A-2004-292427
patent reference 2: JP-A-2003-327533
patent reference 3: JP-A-2005-154431
patent reference 4: JP-A-62-277322
patent reference 5: JP-A-2000-281564
patent reference 6: JP-A-2000-103731
patent reference 7: JP-A-2004-292442
patent reference 8: JP-A-2004-300149
patent reference 9: US Patent No. 6610323
patent reference 10: WO01/51050
patent reference 11: WO03/61584
non-patent reference 1: "DRUG DEVELOPMENT AND INDUSTRIAL PHARMACY", 18(13), 1437-1447 (1992)

### Disclosure of the Invention

### Problems to be Solved by the Invention

There is a demand for the development of a solid preparation comprising an active ingredient having high stability, wherein the active ingredient stably and rapidly expresses its pharmacological effect after administration, and the pharmacological effect is sustained for a prolonged period of time.
The present inventors have conducted intensive studies and found that a solid preparation comprising (1) an antacid, (2) an immediate-release part containing a compound unstable to acid and a basic substance, and (3) a sustained-release part containing a compound unstable to acid and a basic substance and having a film that dissolves at pH 6.5 or above in combination shows high stability of the active ingredient, expresses a pharmacological effect of the active ingredient stably and rapidly after administration, and sustains the pharmacological effect for a prolonged period of time, which resulted in the completion of the present invention.

### Means of Solving the problems

Accordingly, the present invention provides
[1] a controlled release solid preparation comprising (1) an antacid, (2) an immediate-release part comprising a compound unstable to acid and a basic substance, and (3) a sustained-release part containing a compound unstable to acid and a basic substance and having a film that dissolves at pH 6.5 or above in combination;
[2] the preparation of the above-mentioned [1], wherein the film that dissolves at pH 6.5 or above contains one kind or a mixture of two or more kinds selected from the group consisting of hydroxypropyl methylcellulose phthalate, cellulose acetate phthalate, carboxymethylethylcellulose, methyl methacrylate-methacrylic acid copolymer, methacrylic acid-ethyl acrylate copolymer, methacrylic acid-methyl acrylate-methyl methacrylate copolymer, hydroxypropyl methylcellulose acetate succinate, polyvinyl acetate phthalate and shellac;
[3] the preparation of the above-mentioned [1], wherein the sustained-release part comprises a core particle comprising a compound unstable to acid and a basic substance, an intermediate layer formed on the surface of the core particle and a film that dissolves at pH 6.5 or above, which is formed via the intermediate layer;
[4] the preparation of the above-mentioned [1], wherein the compound unstable to acid is a proton pump inhibitor (PPI);
[5] the preparation of the above-mentioned [4], wherein the PPI is a compound represented by the formula (I):

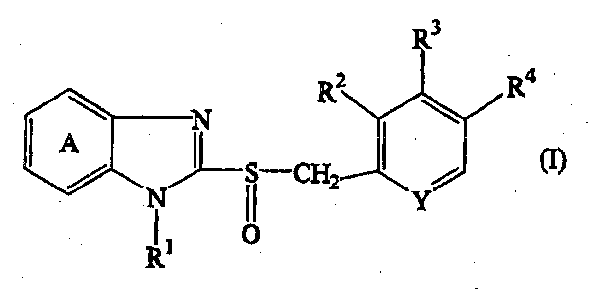

wherein ring A is a benzene ring optionally having
substituent (s), R¹ is a hydrogen atom, an aralkyl group optionally having substituent(s), an acyl group or an acyloxy group, R², R³ and R⁴ are the same or different and each is a hydrogen atom, an alkyl group optionally having substituent(s), an alkoxy group optionally having substituent(s) or an amino group optionally having substituent(s), and Y is a nitrogen atom or CH, or an optically active form thereof or a salt thereof;
[6] the preparation of the above-mentioned [4], wherein the PPI is lansoprazole, omeprazole, rabeprazole, pantoprazole, ilaprazole or an optically active form thereof or a salt thereof;
[7] the preparation of the above-mentioned [1], wherein the antacid is at least one kind of component selected from a metal oxide, a metal hydroxide and an alkaline earth metal carbonate;
[8] the preparation of the above-mentioned [1], wherein a 1% aqueous solution or 1% aqueous suspension of the antacid has a pH of not less than 8.0;
[9] the preparation of the above-mentioned [7], wherein the metal oxide is at least one kind selected from the group consisting of magnesium oxide, magnesium silicate, dry aluminum hydroxide gel and magnesium aluminometasilicate;
[10] the preparation of the above-mentioned [7], wherein the metal hydroxide is at least one kind selected from the group consisting of magnesium hydroxide, aluminum hydroxide, synthetic hydrotalcite, coprecipitate of aluminum hydroxide and magnesium hydroxide, coprecipitate of aluminum hydroxide, magnesium carbonate and calcium carbonate and coprecipitate of aluminum hydroxide and sodium hydrogen carbonate;
[11] the preparation of the above-mentioned [7], wherein the alkaline earth metal carbonate is calcium carbonate or magnesium carbonate;
[12] the preparation of the above-mentioned [1], wherein the content of the antacid is 5 mEq - 50 mEq;
[13] the preparation of the above-mentioned [1], wherein the weight ratio of the contents of the compound unstable to acid in the immediate-release part and the sustained-release part is 10:1 - 1:10;
[14] the preparation of the above-mentioned [1], which shows an increase in the-intragastrie average-pH to 4 or above in 0.5 hr after oral administration to a mammal and a retention time at pH 4 or above of not less than 14 hr a day; and
[15] a solid preparation showing an increase in the intragastric average pH to 4 or above in 0.5 hr after oral administration to a mammal and a retention time at pH 4 or above of not less than 14 hr a day.

The present invention is explained in detail in the following.
The controlled release solid preparation of the present invention comprises (1) an antacid, (2) an immediate-release part containing a compound unstable to acid and a basic substance, and (3) a sustained-release part containing a compound unstable to acid and a basic substance and having a film that dissolves at pH 6.5 or above in combination.

In the present specification, the terms "controlled release solid preparation of the present invention" and "solid preparation of the present invention" are used interchangeably unless otherwise specified.

### (1) Antacid

The solid preparation of the present invention contains an antacid. An antacid neutralizes the intragastric pH prior to the release of a compound unstable to acid, which is the active ingredient, in the stomach, whereby the residual ratio of the compound is increased and a stable and rapid pharmacological effect of the compound can be exhibited. As the antacid to be used in the present invention, at least one kind of component selected from the group consisting of metal oxide, metal hydroxide and alkaline earth metal carbonate is preferable.
As the above-mentioned metal oxide, at least one kind selected from the group consisting of magnesium oxide, magnesium silicate (2MgO·3SiO₂·xH₂O), dry aluminum hydroxide gel (Al₂O₃·xH₂O) and magnesium aluminometasilicate (Al₂O₃·MgO·2SiO₂· xH₂O), all for pharmaceutical agents, is preferably used.
Of these, magnesium oxide is more preferable. Magnesium oxide for medical use, which is superior in acid reactivity and has a neutralizing power, is preferable. As such magnesium oxide, one obtained by a general production method and a commercially available product can be used. What is called light burnt magnesia, which is obtained by calcination at a low temperature, is preferable. One obtained by calcination at a temperature of about 500°C - about 1000°C is generally preferable and, from the aspect of neutralizing power, one obtained by calcination at about 600°C - about 900°C is particularly preferable, and one obtained by calcination at about 800°C is most preferable. Of such magnesium oxides, one having a BET specific surface area of generally 10 - 50 m²/g, preferably 20 - 50 m²/g, is most preferable.
Here, the BET specific surface area is a specific surface area measured by a.nitrogen gas adsorption method, where the specific surface area is measured based on the amount of nitrogen gas adsorbed by a certain amount of the surface of magnesium oxide and fine pores into which the nitrogen gas enters.
Examples of magnesium oxide include commercially available heavy magnesium oxide (manufactured by Kyowa chemical Industries Ltd.), heavy magnesium oxide (manufactured by Tomita Pharmaceutical Co., Ltd.), heavy magnesium N-oxide (manufactured by Kyowa chemical Industries Ltd.), light magnesium oxide (manufactured by Kyowa chemical Industries Ltd.) and the like. Particularly, heavy magnesium N-oxide (manufactured by Kyowa chemical Industries Ltd.) and the like are preferable.

As the metal hydroxide, at least one kind selected from the group consisting of magnesium hydroxide, aluminum hydroxide, synthetic hydrotalcite (Mg₆Al₂ (OH) ₁₆CO₃·4H₂O), coprecipitate of aluminum hydroxide and magnesium hydroxide, coprecipitate of aluminum hydroxide, magnesium carbonate and calcium carbonate, and coprecipitate of aluminum hydroxide and sodium hydrogen carbonate, all for pharmaceutical agents, is preferable. Of these, magnesium hydroxide is preferable in view of the disintegration property of preparation, dissolution property of compound unstable to acid and the like.

As the above-mentioned alkaline earth metal carbonate, calcium carbonate and magnesium carbonate for pharmaceutical agents, and the like can be used.

The above-mentioned metal oxide, metal hydroxide and alkaline earth metal carbonate may be used alone or two or more kinds thereof may be combined.
Some of the metal oxides and metal hydroxides polish surface of a formulating device during production to afford tablets having an entirely or partially dark surface, or a dark spot, line or plane, or attach to a punch for tabletting. These properties markedly reduce the productivity. Thus, it has been found that, when a metal oxide or metal hydroxide having abradability and punch-sticking property is to be selected, a metal oxide and a metal hydroxide free of such properties may be used in combination, or they may be subjected to wet or dry granulation together with an additive usable for pharmaceutical products (e.g., excipient, binder, disintegrant and the like explained in the below-mentioned (4)), whereby the polishing action and punch-sticking property can be suppressed.

The above-mentioned antacid preferably shows a pH of not less than 8.0, more preferably within the range of 8.0 - 12.0, when it is prepared into a 1% aqueous solution or 1% aqueous suspension.

The above-mentioned antacid is added, in an amount permitting rapid dissolution of the antacid to neutralize gastric acid, together with intragastric disintegration of solid preparation, and preferably prior to dissolution of the compound unstable to acid, so as to prevent unstabilization of the compound unstable to acid by exposure to the gastric acid. While the amount varies depending on the ability of each antacid to neutralize gastric acid, it is preferably 5 mEq - 50 mEq, more preferably 10 mEq - 50 mEq, in the solid preparation of the present invention.

### (2) Immediate-release part

The immediate-release part of the solid preparation of the present invention contains a compound unstable to acid and a basic substance.
In the immediate-release part, the release property of a compound unstable to acid, which is the active ingredient, is immediate-release. Here, the immediate-release means an elution ratio of the active ingredient at 30 min after the start of the test of not less than 85% when the Japanese Pharmacopoeia Dissolution Test Method 2 (Paddle Method) is performed using a suitable test solution (500 mL or 900 mL) under the conditions of paddle rotation of 100 rpm. As a test solution of a compound unstable to acid in an immediate-release part, for examples, a test solution showing a concentration of the active ingredient of not more than 1/3 of the saturation solubility of the compound unstable to acid upon 100% dissolution thereof in the test solution is used. Preferably, 2nd fluid of the Japanese Pharmacopoeia Dissolution Test Method, or water is used.

### (2-1) compound unstable to acid

The above-mentioned compound unstable to acid is not particularly limited, and may be any compound that becomes unstable when exposed to gastric acid. As such compound unstable to acid, for example, anti-inflammatory enzyme agents such as PPI, erythromycin antibacterial compounds, serrapeptase, semi-alkaline proteinase and the like, and the like can be used, PPI is preferable.
As PPI, for example, a compound represented by the following formula (I) [hereinafter sometimes to be referred to simply as compound (I)] is preferable.

Examples of compound (I) include a compound represented by the formula (I):

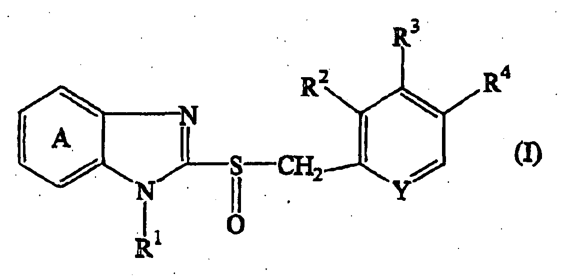

wherein ring A is a benzene ring optionally having substituent(s), R¹ is a hydrogen atom, an aralkyl group optionally having substituent(s), an acyl group or an acyloxy group, R², R³ and R⁴ are the same or different and each is a hydrogen atom, an alkyl group optionally having substituent(s), an alkoxy group optionally having substituent(s) or an amino group optionally having substituent(s), and Y is a nitrogen atom or CH, or an optically active form thereof or a salt thereof.

In the above-mentioned compound (I), examples of the "substituent" of the "benzene ring optionally having substituent(s)" for ring A include a halogen atom, a cyano group, a nitro group, an alkyl group optionally having substituent (s), a hydroxy group, an alkoxy group optionally having substituent(s), an aryl group, an aryloxy group, a carboxy group, an acyl group, an acyloxy group, a 5- to 10-membered heterocyclic group and the like. The benzene ring may be substituted by about 1 to 3 of these substituents. When the number of substituents is two or more, each substituent may be the same or different. Of these substituents, a halogen atom, an alkyl group optionally having substituent(s), an alkoxy group optionally having substituent(s) and the like are preferable.
Examples of the halogen atom include fluorine, chlorine, bromine atom and the like. Of these, a fluorine atom is preferable.
Examples of the "alkyl group" of the "alkyl group optionally having substituent (s)" include a C₁₋₇ alkyl group (e.g., methyl, ethyl, propyl, isopropyl, butyl, isobutyl, sec-butyl, tert-butyl, pentyl, hexyl, heptyl group etc.) and the like. Examples of the "substituent" of the "alkyl group optionally having substituent(s)" include a halogen atom, a hydroxy group, a C₁₋₆ alkoxy group (e.g., methoxy, ethoxy, propoxy, butoxy etc.), a C₁₋₆ alkoxy-carbonyl group (e.g., methoxycarbonyl, ethoxycarbonyl, propoxycarbonyl etc.), a carbamoyl group and the like, and the number of these substituents may be about 1 to 3. When the number of substituents is two or more, each substituent may be the same or different.
Examples of the "alkoxy group" of the "alkoxy group optionally having substituent(s)" include a C₁₋₆ alkoxy group (e.g., methoxy, ethoxy, propoxy, isopropoxy, butoxy, isobutoxy, pentoxy etc.) and the like. Examples of the "substituent" of the "alkoxy group optionally having substituent(s)" include those similar to the "substituent" of the above-mentioned "alkyl group optionally having substituent(s)", and the number of substituents is the same.
Examples of the "aryl group" include a C₆₋₁₄ aryl group (e.g., phenol, 1-naphthyl, 2-naphthyl, biphenyl, 2-anthryl etc.) and the like.
Examples of the "aryloxy group" include a C₆₋₁₄ aryloxy group (e.g., phenyloxy, 1-naphthyloxy, 2-naphthyloxy etc.) and the like.
Examples of the "acyl group" include formyl, alkylcarbonyl, alkoxycarbonyl, carbamoyl, alkylcarbamoyl, alkylsulfinyl, alkylsulfonyl and the like.
Examples of the "alkylcarbonyl group" include a C₁₋₆ alkyl-carbonyl group (e.g., acetyl, propionyl etc.) and the like.
Examples of the "alkoxycarbonyl group" include a C₁₋₆ alkoxy-carbonyl group (e.g., methoxycarbonyl, ethoxycarbonyl, propoxycarbonyl, butoxycarbonyl etc.) and the like.
Examples of the "alkylcarbamoyl group" include an N-C₁₋₆ alkyl-carbamoyl group (e.g., methylcarbamoyl, ethylcarbamoyl group etc.), an N,N-di-C₁₋₆ alkyl-carbamoyl group (e.g., N,N-dimethylcarbamoyl, N,N-diethylcarbamoyl etc.) and the like.
Examples of the "alkylsulfinyl group" include a C₁₋₇ alkylsulfinyl group (e.g., methylsulfinyl, ethylsulfinyl, propylsulfinyl, isopropylsulfinyl etc.) and the like.
Examples of the "alkylsulfonyl group" include a C₁₋₇ alkylsulfonyl group (e.g., methylsulfonyl, ethylsulfonyl, propylsulfonyl, isopropylsulfonyl etc.) and the like.
Examples of the "acyloxy group" include alkylcarbonyloxy, alkoxycarbonyloxy, carbamoyloxy, alkylcarbamoyloxy, alkylsulfinyloxy, alkylsulfonyloxy and the like.
Examples of the "alkylcarbonyloxy group" include a C₁₋₆ alkyl-carbonyloxy group (e.g., acetyloxy, propionyloxy etc.) and the like.
Examples of the "alkoxycarbonyloxy group" include a C₁₋₆ alkoxy-carbonyloxy group (e.g., methoxycarbonyloxy, ethoxycarbonyloxy, propoxycarbonyloxy, butoxycarbonyloxy etc.) and the like.
Examples of the "alkylcarbamoyloxy group" include a C₁₋₆ alkyl-carbamoyloxy group (e.g., methylcarbamoyloxy, ethylcarbamoyloxy etc.) and the like.
Examples of the "alkylsulfinyloxy group" include a C₁₋₇ alkylsulfinyloxy group (e.g., methylsulfinyloxy, ethylsulfinyloxy, propylsulfinyloxy, isopropylsulfinyloxy etc.) and the like.
Examples of the "alkylsulfonyloxy group" include a C₁₋₇ alkylsulfonyloxy group (e.g., methylsulfonyloxy, ethylsulfonyloxy, propylsulfonyloxy, isopropylsulfonyloxy etc.) and the like.
Examples of the "5- to 10-membered heterocyclic group" include a 5- to 10-membered (preferably 5- or 6-membered) heterocyclic group containing, besides carbon atom, one or more (e.g., 1 - 3) hetero atoms selected from nitrogen atom, sulfur atom and oxygen atom and the like. Specific examples include 2- or 3-thienyl group, 2-, 3- or 4-pyridyl group, 2- or 3-furyl group, 1-, 2- or 3-pyrrolyl group, 2-, 3-, 4-, 5- or 8-quinolyl group, 1-, 3-, 4- or 5-isoquinolyl group, 1-, 2- or 3-indolyl group and the like. Of these, preferred is a 5- or 6-membered heterocyclic group such as 1-, 2- or 3-pyrrolyl group and the like.
Preferably, ring A is a benzene ring optionally having 1 or 2 substituents selected from a halogen atom, an optionally halogenated C₁₋₄ alkyl group, an optionally halogenated C₁₋₄ alkoxy group and a 5- or 6-membered heterocyclic group.

Examples of the "aralkyl group" of the "aralkyl group optionally having substituent (s)" for R¹ include a C₇₋₁₆ aralkyl group (e.g., C₆₋₁₀ aryl C₁₋₆ alkyl group such as benzyl, phenethyl etc., and the like) and the like. Examples of the "substituent" of the "aralkyl group optionally having substituent(s)" include substituents similar to the "substituent" of the above-mentioned "alkyl group optionally having substituent(s)", and the number of substituents is about 1 to 4. When the number of substituents is two or more, each substituent may be the same or different.
Examples of the "acyl group" for R¹ include the "acyl group" described as a substituent for the above-mentioned ring A and the like.
Examples of the "acyloxy group" for R¹ include the "acyloxy group" described as a substituent for the above-mentioned ring A and the like.
Preferable R¹ is a hydrogen atom.

Examples of the "alkyl group optionally having substituent(s)" for R², R³ or R⁴ include the "alkyl group optionally having substituent(s)" described as a substituent for the above-mentioned ring A and the like.
Examples of the "alkoxy group optionally having substituent (s)" for R², R³ or R⁴ include the "alkoxy group optionally having substituent(s)" described as the substituent for the above-mentioned ring A and the like.
Examples of the "amino group optionally having substituent (s)" for R², R³ or R⁴ include an amino group, a mono-C₁₋₆ alkylamino group (e.g., methylamino, ethylamino etc.), a mono-C₆₋₁₄ arylamino group (e.g., phenylamino, 1-naphthylamino, 2-naphthylamino etc.), ,a di-C₁₋₆ alkylamino group (e.g., dimethylamino, diethylamino etc.), a di-C₆₋₁₄ arylamino group (e.g., diphenylamino etc.) and the like.
Preferable R² is a C₁₋₆ alkyl group, a C₁₋₆ alkoxy group, a C₁₋₆ alkoxy-C₁₋₆ alkoxy group or a di-C₁₋₆ alkylamino group. More preferable R² is a C₁₋₃ alkyl group or a C₁₋₃ alkoxy group.
Preferable R³ is a hydrogen atom, a C₁₋₆ alkoxy-C₁₋₆ alkoxy group or an optionally halogenated C₁₋₆ alkoxy group. More preferable R³ is a C₁₋₃ alkoxy group which is optionally halogenated or substituted by a C₁₋₃ alkoxy group.
Preferable R⁴ is a hydrogen atom or C₁₋₆ alkyl group. More preferable R⁴ is a hydrogen atom or a C₁₋₃ alkyl group (particularly a hydrogen atom).
Preferable Y is a nitrogen atom.

Preferable compound of the formula (I) is a compound wherein ring A is a benzene ring optionally having substituent(s) selected from a halogen atom, an optionally halogenated C₁₋₄ alkyl group, an optionally halogenated C₁₋₄ alkoxy group and a 5- or 6-membered heterocyclic group, R¹ is a hydrogen atom, R² is a C₁₋₆ alkyl group, a C₁₋₆ alkoxy group, a C₁₋₆ alkoxy-C₁₋₆ alkoxy group or a di-C₁₋₆ alkylamino group, R³ is a hydrogen atom, a C₁₋₆ alkoxy-C₁₋₆ alkoxy group or an optionally halogenated C₁₋₆ alkoxy group, R⁴ is a hydrogen atom or a C₁₋₆ alkyl group, and Y is a nitrogen atom.

Of compound (I), a compound represented by the formula (Ia):

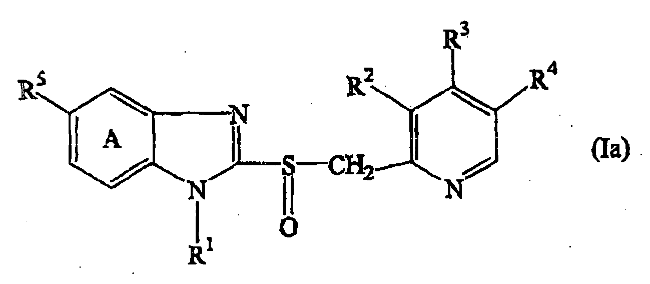

wherein R¹ is a hydrogen atom, R² is a C₁₋₃ alkyl group or a C₁₋₃ alkoxy group, R³ is a C₁₋₃ alkoxy group optionally halogenated or substituted by a C₁₋₃ alkoxy group, R⁴ is a hydrogen atom or a C₁₋₃ alkyl group, and R⁵ is a hydrogen atom, an optionally halogenated C₁₋₃ alkoxy group or a pyrrolyl group (e.g., 1-, 2- or 3-pyrrolyl group).
In the formula (Ia), a compound wherein R¹ is a hydrogen atom, R² is a C₁₋₃ alkyl group, R³ is an optionally halogenated C₁₋₃ alkoxy group, R⁴ is a hydrogen atom, and R⁵ is a hydrogen atom or an optionally halogenated C₁₋₃ alkoxy group is particularly preferable.

Specific examples of compound (I) include the following compounds.
2-[[[3-Methyl-4-(2,2,2-trifluoroethoxy)-2-pyridinyl]methyl]sulfinyl]-1H-benzimidazole, 2-[[(3,5-dimethyl-4-methoxy-2-pyridinyl) methyl]sulfinyl]-5-methoxy-1H-benzimidazole 2-[[[4-(3-methoxypropoxy)-3-methyl-2-pyridinyl]methyl]sulfinyl]-1H-benzimidazole-sodium salt, 5-difluoromethoxy-2 -[[(3,4-dimethoxy-2-pyridinyl)methyl]sulfinyl]-1H-benzimidazole and the like.
Of these compounds, 2-[[[3-methyl-4-(2,2,2-trifluoroethoxy)-2-pyridinyl]methyl]sulfinyl]-1H-benzimidazole(lansoprazole)is preferable.

Compound (I) may be a racemate or an optically active form such as R-form, S-form and the like. For example, compound (I) may be an optically active form such as (R)-2-[[[3-methyl-4-(2,2,2-trifluoxoethoxy)-2-pyridinyl)methyl]sulfinyl]-1H-benzimidazole (to be sometimes referred to as lansoprazole R form) and the like. In addition, the optically active form is preferable.

As a salt of compound (I) or an optically active form thereof, a pharmaceutically acceptable salt is preferable. For example, salts of compound (I) or an optically active form thereof with an inorganic base, an organic base and a basic amino acid, and the like can be mentioned.
Preferable examples of the salt with inorganic base include alkali metal salts such as sodium salt, potassium salt and the like; alkaline earth metal salts such as calcium salt, magnesium salt and the like; ammonium salt and the like.
Preferable examples of the salt with organic base include salts with alkylamine (trimethylamine, triethylamine etc.), heterocyclic amine (pyridine, picoline etc.), alkanolamine (ethanolamine, diethanolamine, triethanolamine etc.), dicyclohexylamine, N,N'-dibenzylethylene diamine and the like.
Preferable examples of the salt with basic amino acid include salts with arginine, lysine, ornithine and the like.
Of these, preferred is an alkali metal salt or an alkaline earth metal salt. Particularly, a sodium salt is preferable.
Compound (I) can be produced by a method known per se, for example, the method described in JP-A-61-50978, US-B-4,62E,098, JP-A-10-195068, WO98/21201 and the like or a method analogous thereto.
The optically active form of compound (I) can be obtained by a method such as an optical resolution method (fractional recrystallization, chiral column method, diastereomer method, a method using microorganism or enzyme etc.), asymmetric oxidation and the like. For example, a lansoprazole R form can be produced according to the methods described in WO00/78745, WO01/83473, WO01/87874 and WO02/44167.

The PPI to be used in the present invention is preferably selected from benzimidazole compounds having an antiulcer activity such as lansoprazole, omeprazole, rabeprazole, pantoprazole and ilaprazole, and optically active forms thereof and pharmaceutically acceptable salts thereof. More preferably, it is lansoprazole, omeprazole, rabeprazole or pantoprazole.

### (2-2) Basic substance

In the present invention, a basic substance is added to the immediate-release part to stabilize the above-mentioned compound unstable to acid in the preparation.
Examples of the above-mentioned basic substance include alkaline earth metal carbonates (e.g., calcium carbonate, magnesium carbonate for pharmaceutical agent etc.), tromethamol, disodium succinate, sodium hydrogenphosphate, trisodium phosphate, dipotassium phosphate, L-arginine and the like. Preferred is alkaline earth metal carbonate, and more preferred is calcium carbonate. These basic substances may be used alone or two or more kinds thereof may be used in combination.
The amount of the basic substance to be added is not particularly limited as long as it is sufficient to stabilize the above-mentioned substance unstable to acid. It is generally 1.0 wt% - 60 wt%, preferably 3.0 wt% - 50 wt%, relative to the total amount of the immediate-release part.
The above-mentioned basic substance should be distinguished from the antacid explained in the above-mentioned (1). For example, a substance used as the above-mentioned basic substance may also be used as the antacid of the above-mentioned (1) (e.g., the above-mentioned "alkaline earth metal carbonate"). When such substance is used as the antacid, it neutralizes the intragastric pH. On the other hand, when it is added to the immediate-release part as a basic substance, it stabilizes a compound unstable to acid in the preparation.

The form of the above-mentioned immediate-release part may be any. To achieve immediate release, granules, fine granules and the like are preferable.
The above-mentioned immediate-release part can be produced by a method known per se. For example, it can be produced by combining adequate amounts of a compound unstable to acid and a basic substance and, where necessary, an additive such as excipient, binder, disintegrant, lubricant, corrigent, colorant, flavor and the like, and granulating the mixture.
The above-mentioned granulation is preferably performed by a wet granulation method. The wet granulation method comprises dispersing or dissolving a mixture of the active ingredient and other components such as excipient and the like in water, a binder or a solvent, granulating the dispersion or solution, and drying same to give a granulation product such as granules, fine granules and the like. The wet granulation method can be performed according to a method known in the pharmaceutical field. As the granulation mechanism, for example, known methods such as extrusion, fluidizing, tumbling, centrifugation, stirring, spraying and the like can be used.

### (3) Sustained-release part

The sustained-release part in the solid preparation of the present invention contains a compound unstable to acid and a basic substance and has a film that dissolves at pH 6.5 or above.
In the sustained-release part, the release property of a compound unstable to acid, which is the active ingredient, is sustained-release. The sustained-release means an elution ratio of the active ingredient at 30 min after the start of the test of less than 85% when the Japanese Pharmacopoeia Dissolution Test Method 2 (Paddle Method) is performed using a suitable test solution (500 mL or 900 mL) under the conditions of paddle rotation of 100 rpm. As the test solution here, those similar to the ones recited in the explanation of the above-mentioned (2) Immediate-release part can be used.

### (3-1) compound unstable to acid

As the above-mentioned compound unstable to acid, those similar to the compounds unstable to acid recited in the explanation of the above-mentioned (2-1)'can be used, with preference given to PPI. The compound unstable to acid contained in the sustained-release part may be the same as or different from the compound unstable to acid contained in the immediate-release part.

### (3-2) basic substance

In the present invention, a basic substance is added to the sustained-release part to stabilize the compound unstable to acid in the preparation.
As the above-mentioned basic substance, those similar to the basic substances explained in the above-mentioned (2-2) can be mentioned, and magnesium carbonate is preferable. The basic substance to be contained in the sustained-release part may be the same as or different from the basic substance to be contained in the immediate-release part.

### (3-3) film that dissolves at pH 6.5 or above

The sustained-release part in the solid preparation of the present invention has a film that dissolves at pH 6.5 or above.
The film that dissolves at pH 6.5 or above is not particularly limited as long as it dissolves at pH 6.5 or above, preferably at pH not less than 6.5 and not more than 7.5, more preferably at pH not less than 6.5 and not more than 7.0. It preferably contains a mixture of one or more kinds selected from the group consisting of hydroxypropyl methylcellulose phthalate, cellulose acetate phthalate, carboxymethylethylcellulose, methyl methacrylate-methacrylic acid copolymer, methacrylic acid-ethyl acrylate copolymer, methacrylic acid-methyl acrylate-methyl methacrylate copolymer, hydroxypropyl methylcellulose acetate succinate, polyvinyl acetate phthalate and shellac.

Examples of the hydroxypropyl methylcellulose phthalate include HPMCP(HP-55) (trade name, manufactured by Shin-Etsu Chemical Co., Ltd.), HPMCP(HP-50) (trade name, manufactured by Shin-Etsu Chemical Co., Ltd.) and the like.

Examples of the carboxymethylethylcellulose include CMEC (trade name, manufactured by Freund Corporation) and the like.

Examples of the methyl methacrylate-methacrylic acid copolymer include Eudragit L100 (trade name, manufactured by Rohm), Eudragit S100 (trade name, manufactured by Rohm) and the like.

Examples of the methacrylic acid-ethyl acrylate copolymer include Eudragit L100-55 (trade name, manufactured by Rohm), Eudragit L30D-55 (trade name, manufactured by Rohm) and the like.

. Examples of the methacrylic acid-methyl acrylate-methyl methacrylate copolymer include Eudragit FS30D (trade name, manufactured by Rohm) and the like.

Examples of the hydroxypropyl methylcellulose acetate succinate include AQOAT AS-L (trade name, manufactured by Shin-Etsu Chemical Co., Ltd.), AQOAT AS-M (trade name, manufactured by Shin-Etsu Chemical Co., Ltd.), AQOAT AS-H (trade name, manufactured by Shin-Etsu Chemical Co., Ltd.) and the like.

The above-mentioned materials of the film are used alone or a combination of two or more kinds so that the obtained film will dissolve at pH 6.5 or above, preferably at pH not less than 6.5 and not more than 7.5, more preferably at pH not less than pH 6.5 and not more than 7.0. Alternatively, two or more kinds of the materials may be successively applied to give a film having a multi-layer structure.
Alternatively, a polymer that dissolves at pH 6.0 or above and a polymer that dissolves at pH 7.0 or above may be appropriately combined, so that the resulting film will dissolve at pH 6.5 or above, preferably at pH not less than 6.5 and not more than 7.5, more preferably at pH not less than pH 6.5 and not more than 7.0. In this case, the polymer that dissolves at pH 6.0 or above and the polymer that dissolves at pH 7.0 or above are desirably combined at a ratio of 1:0.5 - 1:5 and used.

The above-mentioned film may contain, where necessary, a plasticizer, a stabilizer and the like, such as polyethylene glycol, dibutyl sebacate, ethyl phthalate, triacetine, triethyl citrate and the like.
While the amount of the above-mentioned materials for the film is not particularly limited, it is preferably 5% - 200%, more preferably 20% - 100%, most preferably 30% - 60%, relative to the core particle.
The above-mentioned "having" film includes having not only a film-like coating but also a coating with a higher thickness, and further, not only a cover film on the entire surface of a core particle containing a compound unstable to acid and a basic substance but also a cover film on a part of the surface of the core particle (e.g., cover film on most of the surface (not less than 80%) of the core particle, though partially uncoated).

While the form of the above-mentioned sustained-release part is not particularly limited, it preferably has a form comprising a core particle containing a compound unstable to acid'and a basic substance and a film that dissolves at pH 6.5 or above, which is formed on the surface of the particle.
As the core of such sustained-release part, tablet, granules or fine granules comprising an inactive carrier [e.g., Nonpareil (Nonpareil-101 (particle size 850-710, 710-500, 500-355), Nonpareil-103 (particle size 850-710, 710-500, 500-355), Nonpareil-105 (particle size 300-180), manufactured by Freund Industry Co., Ltd.), Celphere (CP-507 (particle size 500-710), CP-305 (particle size 300-500), CP-203 (particle size 150-300), CP-102 (particle size 106-212) , SCP-100 (particle size 75-212), manufactured by Asahi Kasei Chemicals Co., Ltd.) etc.] as a core, and a coating liquid containing a substance unstable to acid and a basic substance, which is applied to the surface of the core; a tablet prepared using the granules or fine granules; particles obtained by granulating the active ingredient and an excipient generally used for preparation making and the like can be used.
The core particle can be produced, for example, by the method described in JP-A-63-30181,6. For example, when a core particle is to be obtained by applying the above-mentioned coating solution on the core of the inactive carrier, a core particle containing a substance unstable to acid and a basic substance can be prepared by wet granulation using a rotating fluidized bed coater (SPIR-A-FLOW, manufactured by Freund Industry Co., Ltd.), a centrifugal fluid bed granulator (CF-mini, CF-360, manufactured by Freund Industry Co., Ltd.), a rotating fluidized bed granulator (POWREX MP-10) and the like. Alternatively, the above-mentioned coating solution may be sprayed to form a coating while spraying a solution containing a binder and the like on the core of the inactive carrier. In this case, the production apparatus is not limited. However, a centrifugal fluid bed granulator and the like is preferably used. Alternatively, coating by the above-mentioned two kinds of apparatuses may be combined and the substance unstable to acid and a basic substance may be applied in two steps.
Alternatively, the core particle may be prepared by dry granulation using a roller compactor and the like.
On the other hand, when an inactive carrier core is not used, a core particle containing a compound unstable to acid and basic substance can be obtained by adding a compound unstable to acid and a basic substance and, where necessary, an excipient such as lactose, sucrose, mannitol, cornstarch, crystalline cellulose and the like to a binder such as hydroxypropylmethylcellulose, hydroxypropylcellulose, methylcellulose, polyvinyl alcohol, macrogol, pluronic F68, gum arabic, gelatin, starch and the like and, where necessary, a disintegrant such as sodium carboxymethylcellulose, calcium carboxymethylcellulose, sodium croscarboxymethylcellulose (Ac-Di-Sol, manufactured by FMC International), polyvinylpyrrolidone, low-substituted hydroxypropylcellulose and the like, and granulating the mixture by a stirring granulator, wet extrusion-granulator, fluid bed granulator and the like.
The obtained core particle is sieved as necessary to give a particle having a desired particle size. While the particle, size is not particularly limited, it is generally about 50 µm - about 5 mm, preferably about 100 µm - about 3 mm, more preferably about 100 µm - about 2 mm.

In the above-mentioned sustained-release part, an intermediate layer is more preferably formed between a core particle containing a compound unstable to acid and a basic substance, and a film that dissolves at pH 6.5 or above. By shutting off a direct contact of a core particle with a film by an intermediate layer, the stability of a compound unstable to acid, which is the active ingredient, can be improved.
Examples of the material for the above-mentioned intermediate layer include a blend of a polymer substrate such as low-substituted hydroxypropylcellulose, hydroxypropylcellulose, hydroxypropylmethylcellulose (e.g., TC-5 and the like), polyvinylpyrrolidone, polyvinyl alcohol, methylcellulose, hydroxyethylmethylcellulose and the like, and saccharides such as sucrose [purified sucrose (pulverized (powder sugar) or unpulverized) and the like], starch sugar such as cornstarch and the like, lactose, honey and sugar alcohol (D-mannitol, erythritol etc.) and the like at an appropriate ratio and the like. Where necessary, moreover, the intermediate layer may contain an excipient (e.g., masking agent (titanium oxide etc.), an antistat (titanium oxide, talc etc.)) and the like as appropriate.
The amount of coating of the intermediate layer is generally about 0.02 part by weight - about 1.5 parts by weight, preferably about 0.05 - about 1 part by weight, relative to 1 part by weight of the core particle. The intermediate layer can be applied by a conventional method. For example, the components of the intermediate layer are preferably diluted with purified water and the like, and sprayed as a liquid. In this case, it is more preferable to apply the liquid while spraying a binder such as hydroxypropylcellulose and the like during coating.
The intermediate layer may consist of a single layer or plural layers.

### (4) Additive

In the solid preparation of the present invention, examples of the additive that may be contained in the immediate-release part and/or sustained-release part as necessary include excipients (e.g., glucose, fructose, lactose, saccharose, D-mannitol, erythritol, maltitol, trehalose, sorbitol, cornstarch, potato starch, wheat starch, rice starch, microcrystalline cellulose, silicic anhydride, anhydrous calcium phosphate, precipitated calcium carbonate, calcium silicate, and the like); binders (e.g., polyvinylpyrrolidone, polyvinyl alcohol, partly pregelatinized starch, pregelatinized starch, sodium alginate, pullulan, gum arabic powder, gelatin etc.); disintegrants (e.g., low-substituted hydroxypropylcellulose, carmellose, carmellose calcium, sodium carboxymethyl starch, croscarmellose sodium, crospovidone, hydroxypropyl starch etc.); corrigents (e.g., citric acid, ascorbic acid, tartaric acid, malic acid, aspartame, acesulfame potassium, thaumatin, sodium saccharin, dipotassium glycyrrhizinate, sodium glutamate, sodium 5'-inosinate, sodium 5'-guanylate etc.); surfactants (e.g., polysorbate, polyoxyethylene-polyoxypropylene copolymer, sodium lauryl sulfate etc.); flavors (e.g., lemon oil, orange oil, menthol, peppermint etc.); lubricants (e.g., magnesium stearate, sucrose esters of fatty acids, sodium stearyl fumarate, stearic acid, talc, polyethylene glycol etc.); colorants (e.g., Food Color Yellow No. 5, Food Color Blue No. 2, diiron trioxide, yellow ferric oxide etc.); antioxidants (e.g., sodium ascorbate, L-cysteine, sodium sulfite etc.) and the like. While the particle size of these additives is not particularly limited, it is preferably not more than 500 µm in view of the particle productivity and easy administration.

### (5) Production method of solid preparation

The solid preparation of the present invention can be obtained by combining the above-mentioned antacid, immediate-release part and sustained-release part. The solid preparation of the present invention can be produced by any method known in the pharmaceutical field. For example, the solid preparation of the present invention can be obtained as fine granules or granules by mixing the antacid, immediate-release part and a sustained-release part by a method known in the pharmaceutical field. In addition, the solid preparation of the present invention can be obtained as a tablet by further punching the solid preparation (fine granules or granules) by a method known in the pharmaceutical field. Alternatively, the solid preparation of the present invention may also be obtained as a capsule by filling the solid preparation (fine granules or granules) into a capsule.

While the total amount of the compound unstable to acid in the solid preparation of the present invention varies depending on the kind, dose and the like of the compound unstable to acid, it is generally 1.0 wt% - 60 wt%, preferably 10 wt% - 50 wt%, more preferably 10 wt% - 40 wt%, of the total amount of the solid preparation of the present invention.
In the solid preparation of the present invention, moreover, the content-weight ratio of the compound unstable to acid in the immediate-release part and the sustained-release part is preferably 10:1 - 1:10, more preferably 5:1 - 1:5, most preferably 2:1 - 1:5.

### (6) Applicable disease, administration mode, dose and the like of the solid preparation of the present invention

When the solid preparation of the present invention contains a PPI such as a compound represented by the formula (I) as a compound unstable to acid, the compound is superior in the antiulcer activity, gastric acid secretion-inhibitory action, mucosa-protecting action, anti-Helicobacter pylori activity and the like, and shows low toxicity. Hence, the solid preparation of the present invention is useful as a pharmaceutical agent. In this case, the solid preparation of the present invention can be orally administered to a mammal (e.g., human, monkey, sheep, horse, dog, cat, rabbit, rat, mouse and the like) for the prophylaxis or treatment of peptic ulcer (e.g., gastric ulcer, duodenal ulcer, anastomotic ulcer, Zollinger-Ellison syndrome and the like), gastritis, GERD (Gastroesophageal Reflux Diseases; erosive esophagitis, esophageal reflux unaccompanied by esophagitis (Symptomatic GERD) and the like), NUD (Non Ulcer Dyspepsia), gastric cancer (including gastric cancer associated with promotion of interleukin-1β production by genetic polymorphism of interleukin-1), stomach MALT lymphoma and the like, eradication or as an aid for eradication of Helicobacter pylori, suppression of peptic ulcer, acute stress ulcer and hemorrhagic stomach, upper gastrointestinal hemorrhage due to invasive stress (stress caused by major surgery requiring postoperative intensive management or cerebrovascular disorder, head trauma, multiple organ failure or extensive burn requiring intensive treatment), prophylaxis or treatment of ulcer caused by non-steroidal anti-inflammatory agent; prophylaxis or treatment of hyperacidity and ulcer due to postoperative stress and the like. For eradication or aid of eradication of Helicobacter pylori, a combined use of the solid preparation of the present invention and a penicillin antibiotic (e.g., amoxicillin and the like) and an erythromycin antibiotic (e.g., clarithromycin and the like) is preferable.
The solid preparation of the present invention is particularly preferably applied as an agent for the prophylaxis or treatment of GERD (Symptomatic GERD, erosive esophagitis and the like).
The solid preparation of the present invention can be directly administered orally. In addition, it is possible to disperse or dissolve the preparation in water, juice, yogurt and the like in advance, and administer the dispersion or solution in the form of a liquid or a semisolid.
The daily dose of the solid preparation of the present invention varies depending on the severity of symptom, the age, sex and body weight of the subject of administration, the timing and interval of administration, the kind of the active ingredient and the like, and is not particularly limited. For example, for oral administration, the dose of the active ingredient of a therapeutic drug for erosive esophagitis (GERD) is about 10 - 200 mg/day, preferably about 30 - 120 mg/day, for an adult (60 kg). The solid preparation of the present invention may be administered once a day or in 2 or 3 portions a day.

The solid preparation of the present invention obtained as mentioned above preferably shows an increase in the intragastric average pH to not less than 4 in about 0.5 hr after administration to a mammal, and the time of retention at pH 4 or above for one day of not less than 14 hr.

The absorption of the compound'unstable to acid in the solid preparation of the present invention from the gastrointestinal tract is controlled by two kinds of systems utilizing the immediate release property of the compound unstable to acid in the immediate-release part and the sustained release property (prolongation of dwelling in the gastrointestinal tract) of the compound unstable to acid in the sustained-release part. When the solid preparation of the present invention is orally administered, the compound unstable to acid is released from the immediate-release part in the stomach immediately after administration, and rapidly exhibits a pharmacological effect. At this time, since an antacid is released in the stomach prior to the release of the compound unstable to acid, decomposition of the compound unstable to acid by gastric acid is suppressed, and the pharmacological effect can be exhibited rapidly and stably. In the meantime, the compound unstable to acid in the sustained-release part is not released in the stomach, gradually moves in the gastrointestinal tract while being protected by a film that dissolves at pH 6.5 or above, and when it reaches the gastrointestinal tract with pH of 6.5 or above (e.g., jejunum, ileum and the like), the film starts to dissolve, and the active ingredient is released from the sustained-release part in a controlled manner. As a result, the solid preparation of the present invention can afford both a rapid pharmacological effect after administration and a pharmacological effect sustained for a prolonged period of time. In addition, since the solid preparation of the present invention contains a basic substance in the immediate-release part and the sustained-release part, the preparation is superior in the stability during the production and preservation.
Accordingly, the solid preparation of the present invention is useful as various preparations for oral administration.

### (Examples)

The present invention is explained in more detail in the following by referring to Preparation Examples, Examples and Experimental Examples, which are not to be construed as limitative.

### Preparation Example 1

### Production of immediate-release part granulated powder

Lansoprazole (hereinafter to be referred to as compound A; 10 g), calcium carbonate (166.67 g) and D-mannitol (155.8 g) were charged in a fluid bed granulator, the mixture was granulated while spraying an aqueous solution of hydroxypropylcellulose (13.87 g) in purified water (231.11 g), and the granules were dried to give a granulated powder (340 g) for the immediate-release part.

### Preparation Example 2

### Production of antacid-containing granulated powder

Magnesium hydroxide (96.67 g), magnesium oxide (133.33 g), D-mannitol (121.87 g) and crospovidone (10.68 g) were charged in a fluid bed granulator, the mixture was granulated while spraying an aqueous solution of hydroxypropylcellulose (13.42 g) in purified water (223.67 g), and the granules were dried to give a granulated powder (370 g) containing an antacid.

### Preparation Example 3

### Preparation of core particle

Core particles to be the core of the sustained-release part were prepared as follows. Hydroxypropylcellulose (HPC-SL, 50 g) was dissolved in purified water (640 g), and low-substituted hydroxypropylcellulose (L-HPC-32W, 25 g) and magnesium carbonate (50 g) were added to the solution and dispersed therein. Compound A (150 g) was uniformly dispersed in the obtained dispersion to give a coating solution. Lactose crystalline cellulose particles (Nonpareil 105, 100 g) were coated with this compound A-containing coating solution (610 g) using a rotating fluidized bed coater (SPIR-A-FLOW, manufactured by Freund Industry Co-, Ltd.). The coating conditions were inlet air temperature: about 60°C, spray air pressure: about 1 kgf/cm², exhaust air gauge :100, BED pressure: about 250 mmHg, rotation speed of rotor :about 300 rpm, spray rate :about 6 g/min, and spray gun position: lower side. After the completion of coating operation, the obtained fine granule was vacuum-dried at 40°C for 16 hr, and sieved using a round sieve to give a core particle having 125 µm 500 µm of particle size.

**[Table 1]**

| [Composition in core particle 85 mg] | |
|---|---|
| lactose crystalline cellulose particle | 30 mg |
| (Nonpareil 105) | |
| compound A | 30 mg |
| magnesium carbonate | 10 mg |
| low-substituted hydroxypropylcellulose | 5 mg |
| hydroxypropylcellulose | 10 mg |
| total | 85 mg |

### Preparation Example 4

The fine particles obtained in Preparation Example 3 were coated with an intermediate layer coating solution using a rotating fluidized bed coater (SPIR-A-FLOW, manufactured by Freund Industry Co., Ltd.), and directly dried to give fine granules with the following composition. The intermediate layer coating solution was produced by dissolving hydroxypropylmethylcellulose 2910 (23.6 g) in purified water (425.5 g) and dispersing titanium oxide (14.2 g) and talc (9.5 g) in the obtained solution. The coating conditions were inlet air temperature: about 60°C, spray air pressure: about 1 kgf/cm², exhaust air gauge: 100, BED pressure :about 250 mmHg, rotation speed of rotor: about 300 rpm, spray rate: about 2.5 g/min and spray gun position : lower side. After the completion of - coating operation, the obtained fine granules were vacuum-dried at 40°C for 16 hr, and sieved using a round sieve to give intermediate layer-coated fine granules having 125 µm - 500 µm of particle size.

**[Table 2]**

| [Composition of intermediate layer-coated fine granules | |
|---|---|
| (191.26mg)] | |
| fine granules obtained in Preparation | 170 mg |

| Example 3 | |
|---|---|
| hydroxypropylmethylcellulose 2910 | 10.63 mg |
| talc | 4.25 mg |
| titanium oxide | 6.38 mg |
| total | 191.26 mg |

### Preparation Example 5

### Preparation of enteric fine granules

Methacrylic acid copolymer S (95.7 g), methacrylic acid copolymer L (31.9 g) and triethyl citrate (12.72 g) were dissolved in a mixed solution of purified water (183.7 g) and anhydrous ethanol (1564 g), and talc (63.8 g) was dispersed in the obtained solution to give a coating solution. The fine granules obtained in Preparation Example 4 were coated with the above-mentioned coating solution using a rotating fluidized bed coater (SPIR-A-FLOW, manufactured by Freund Corporation). A release controlling film that dissolves pH-dependently (releases the active ingredient in an environment with a pH at or above a given level) was applied under the coating conditions of inlet air temperature: about 60°C, spray air pressure: about 1 kgf/cm², exhaust air gauge: 100, BED pressure: about 250 mmHg, rotation speed of rotor; about 150 rpm, spray rate: about 3.0 g/min and spray gun position: lower side. The obtained fine granules were sieved using a round sieve to give enteric fine granules having 125 µm - 500 µm of particle size. The obtained fine granules were-vacuum-dried at 40°C for 16 hr, and the fine granules (220 g) was mixed with talc (0.105 g) and aerosil (0.105 g) to give enteric fine granules.

**[Table 3]**

| [Composition of enteric fine granules (283.3 mg)] | |
|---|---|
| fine granules in Preparation | 191.26 mg |

| Example 4 | |
|---|---|
| methacrylic acid copolymer S | 43.04 mg |
| methacrylic acid copolymer L | 14.36 mg |
| talc | 28.68 mg |
| triethyl citrate | 5.72 mg |
| talc | 0.135 mg |
| aerosil | 0.135 mg |
| total | 283.3 mg |

### Preparation Example 6

### preparation of enteric fine granules

Methacrylic acid copolymer S (127.6 g) and triethyl citrate (12.72 g) were dissolved in a mixed solution of purified water (183.7 g) and anhydrous ethanol (1564 g), and talc (63.8 g) was dispersed in the obtained solution to give a coating solution. The fine granules obtained in Preparation Example 4 were coated with the above-mentioned coating solution using a rotating fluidized bed coater (SPIR-A-FLOW, manufactured by Freund Corporation), A release controlling film that dissolves pH-dependently (releases the active ingredient in an environment with a pH at or above a given level) was applied under the coating conditions of inlet air temperature: about 60°C, spray air pressure: about 1 kgf/cm², exhaust air gauge: 100, BED pressure: about 250 mmHg, rotation speed of rotor: about 150 rpm, spray rate: about 3.0 g/min and spray gun position: lower side. The obtained fine granules were sieved using a round sieve to give enteric fine granules having 125 µm - 500 µm of particle size. The obtained fine granules were vacuum-dried at 40°C for 16 hr, and the fine granules (220 g) was mixed with talc (0.105 g) and aerosil (0.105 g) to give enteric fine granules.

**[Table 4]**

| [Composition of enteric fine granules (283.3 mg)] | |
|---|---|
| fine granules of Preparation | 191.26 mg |

| Example 4 | |
|---|---|
| methacrylic acid copolymer S | 57.40 mg |
| talc | 28.68 mg |
| triethyl citrate | 5.72 mg |
| talc | 0.135 mg |
| aerosil | 0.135 mg |
| total | 283.3 mg |

### Preparation Example 7

### Preparation of core particle

Core particles to be the core of the sustained-release part were prepared as follows. Hydroxypropylmethylcellulose (TC-5EW, 50 g) was dissolved in purified water (640 g), and low-substituted hydroxypropylcellulose (L-HPC-32W, 25 g) and magnesium carbonate (50 g) were added to the solution and dispersed therein. Compound A (150 g) was uniformly dispersed into the obtained dispersion to give a coating solution. Lactose-crystalline cellulose particles (Nonpareil 105T, 130 g) were coated with this compound A-containing coating solution (793 g) using a rotating fluidized bed coater (SPIR-A-FLOW, manufactured by Freund Industry Co., LTd.). The coating conditions were inlet air temperature: about 40°C, spray air pressure: about 1 kgf/cm², exhaust air gauge: 100, BED pressure: about 250 mmHg, rotation speed of rotor: about 300 rpm, spray rate: about 6 g/min and spray gun position: lower side- After the completion of coating operation, the obtained-fine granules were vacuum-dried at 40°C for 16 hr, and sieved using a round sieve to give a core particle having 125 µm - 500 µm of particle size.

**[Table 5]**

| [Composition of core particle (85 mg)] | |
|---|---|
| lactose crystalline cellulose particle | 30 mg |
| (Nonpareil 105T) | |
| compound A | 30 mg |
| magnesium carbonate | 10 mg |
| low-substituted hydroxypropylcellulose | 5 mg |
| hydroxypropylmethylcellulose | 10 mg |
| total | 85 mg |

### Preparation Example 8

The fine granules obtained in Preparation Example 7 were coated with an intermediate layer coating solution using a rotating fluidized bed coater (SPIR-A-FLOW, manufactured by Freund Industry Co., Ltd.), and directly dried to give fine granules of the following composition. The intermediate layer coating solution was produced by dissolving hydroxypropylmethylcellulose 2910 (23.6 g) in purified water (425.5 g) and dispersing titanium oxide (14.2 g) and talc (9.5 g) in the obtained solution. The coating conditions were inlet air temperature: about 60°C, spray air pressure: about 1 kgf/cm², exhaust air gauge: 100, BED pressure: about 250 mmHg, rotation speed of rotor: about 300 rpm, spray rate: about 2.5 g/min and spray gun position: lower side. After the completion of coating operation, the obtained fine granules were vacuum-dried at 40°C for 16 hr, and sieved using a round sieve to give intermediate layer-coated fine granules having 125 µm - 500 µm of particle size.

**[Table 6]**

| [Composition of intermediate layer-coated fine granules | |
|---|---|
| (191.26 mg)] | |
| fine granules obtained in Preparation | 170 mg |

| Example 7 | |
|---|---|
| hydroxypropylmethylcellulose 2910 | 10.63 mg |
| talc | 4.25 mg |
| titanium oxide | 6.38, mg |
| total | 191.26 mg |

### Preparation Example 9

### Preparation of enteric fine granules

Methacrylic acid copolymer S (95.7 g), methacrylic acid copolymer L (31.9 g) and triethyl citrate (12.72 g) were dissolved in a mixed solution of purified water (183.7 g) and anhydrous ethanol (1564 g), talc (63.8 g) was dispersed in the obtained solution to give a coating solution. Fine granules obtained in Preparation Example 8 were coated with the above-mentioned coating solution using a rotating fluidized bed coater (SPIR-A-FLOW, manufactured by Freund Industry Co., Ltd.). A release controlling film that dissolves pH-dependently (releases the active ingredient in an environment with a pH at or above a given level) was applied under the coating conditions of inlet air temperature: about 60°C, spray air pressure: about 1 kgf/cm², exhaust air gauge: 100, BED pressure: about 250 mmHg, rotation speed of rotor: about 150 rpm, spray rate: about 3.0 g/min and spray gun position: lower side. The obtained fine granules were sieved using a round sieve to give enteric fine granules having 125 µm - 500 µm of particle size. The obtained fine granules were vacuum-dried at 40°C for 16 hr, and the fine granules (220 g) was mixed with talc (0.105 g) and aerosil (0.105 g) to give enteric fine granules.

**[Table 7]**

| [Composition of enteric fine granules (283.3 mg)] | |
|---|---|
| fine granules obtained in Preparation | 191.26 mg |

| Example 8 | |
|---|---|
| methacrylic acid copolymer S | 43.04 mg |
| methacrylic acid copolymer L | 14.36 mg |
| talc | 24.68 mg |
| triethyl citrate | 5.72 mg |
| talc | 0.135 mg |
| aerosil | 0.135 mg |
| total | 283.3 mg |

### Preparation Example 10

### Preparation of enteric fine granules

Methacrylic acid copolymer S (127.6 g) and triethyl citrate (12.72 g) were dissolved in a mixed solution of purified water (183.7 g) and anhydrous ethanol (1564 g), talc (63.8 g) was dispersed in the obtained solution to give a coating solution. Fine granules obtained in Preparation Example 8 were coated with the above-mentioned coating solution using a rotating fluidized bed coater (SPIR-A-FLOW, manufactured by Freund Industry Co., Ltd.). A release controlling film that dissolves pH-dependently (releases the active ingredient in an environment with a pH at or above a given level) was applied under the coating conditions of inlet air temperature: about 60°C, spray air pressure: about 1 kgf/cm², exhaust air gauge: 100, BED pressure: about 250 mmHg, rotation speed of rotor: about 150 rpm, spray rate: about 3.0 g/min and spray gun position: lower side. The obtained fine granules were sieved using a round sieve to give enteric fine granules having 125 µm - 500 µm of particle size. The obtained fine granules were vacuum-dried at 40°C for 16 hr, and the fine granules (220 g) was mixed with talc (0.105 g) and aerosil (0.105 g) to give enteric fine granules.

**[Table 8]**

| [Composition of enteric fine granules (283.3 mg)] | |
|---|---|
| fine granules obtained in Preparation | 191.26 mg |

| Example 8 | |
|---|---|
| methacrylic acid copolymer S | 57.40 mg |
| talc | 28.68 mg |
| triethyl citrate | 5.72 mg |
| talc | 0.135 mg |
| aerosil | 0.135 mg |
| total | 283.3 mg |

### Example 1

### Preparation of solid preparation (tablet)

The granulated powder (51.95 g) for the immediate-release part obtained in Preparation Example 1, the antacid-containing granulated powder (37.60 g) obtained in Preparation Example 2, crystalline cellulose (trade name: Ceolus KG-801, manufactured by Asahi Kasei Chemicals, 10.96 g), crospovidone (3.93 g) and magnesium stearate (1.61 g) were mixed in a mortar to give a mixed powder. This mixed powder (10.6 g) and the sustained-release part enteric fine granules (2.13 g) prepared in Preparation Example 9 were gently mixed to give a mixed powder. The obtained mixed powder (1273 mg) was charged in a 14 mmφ flat-faced die with curved edge, from which the solid preparation of the present invention (tablet, 1273 mg) containing compound A (60 mg) was prepared using Autograph (trade name, manufactured by Shimazu Co. Ltd., tabletting pressure: 1 ton/cm²). The obtained tablet showed no darkening.

### Example 2

### Preparation of solid preparation (tablet)

The granulated powder (51.95 g) for the immediate-release part obtained in Preparation Example 1, the antacid-containing granulated powder (37.60 g) obtained in the above-mentioned Preparation Example 2, crystalline cellulose (trade name: Ceolus KG-801, manufactured by Asahi Kasei Chemicals, 10.96 g), crospovidone (3.93 g) and magnesium stearate (1.61 g) were mixed in a mortar to give a mixed powder. This mixed powder (10.6 g) and the enteric fine granules (2.13 g) for the sustained-release part prepared in Preparation Example 10 were mixed in a mortar to give a mixed powder. The obtained mixed powder (1273 mg) was charged in a 14 mmφ flat-faced die with curved edge, from which the solid preparation of the present invention (tablet, 1273 mg) containing compound A (60 mg) was prepared using Autograph (trade name, manufactured by Shimazu Co. Ltd., tabletting pressure: 1 ton/cm²). The obtained tablet showed no darkening.

### Industrial Applicability

According to the present invention, a solid preparation showing high stability of the active ingredient (compound unstable to acid), which expresses a pharmacological effect of the active ingredient stably and rapidly after administration, and sustains the pharmacological effect for a prolonged period of time can be provided. Accordingly, the solid preparation of the present invention is useful as various preparations for oral administration. Particularly, when the compound unstable to acid is a PPI, the solid preparation of the present invention can be useful for the prophylaxis or treatment of peptic ulcer (e.g., gastric ulcer, duodenal ulcer, anastomotic ulcer, Zollinger-Ellison syndrome and the like), gastritis, GERD (Gastroesophageal Reflux Diseases; erosive esophagitis, esophageal reflux unaccompanied by esophagitis (Symptomatic GERD) and the like), NUD (Non Ulcer Dyspepsia), gastric cancer (including gastric cancer associated with promotion of interleukin-1β production by genetic polymorphism of interleukin-1), stomach MALT lymphoma and the like, eradication or as an aid for eradication of Helicobacter pylori, suppression of peptic ulcer, acute stress ulcer and hemorrhagic stomach, upper gastrointestinal hemorrhage due to invasive stress (stress caused by major surgery requiring postoperative intensive management or cerebrovascular disorder, head trauma, multiple organ failure or extensive burn requiring intensive treatment), prophylaxis or treatment of ulcer caused by non-steroidal anti-inflammatory agent; prophylaxis or treatment of hyperacidity, ulcer and the like due to postoperative stress and the like.

This application is based on application No. 2005-378061 filed in Japan, the contents of which are incorporated hereinto by reference. The patent references and non-patent references cited herein are hereby incorporated in full by reference, to the extent that they have been disclosed herein.

## Claims

1. A controlled release solid preparation comprising (1) an antacid, (2) an immediate-release part comprising a compound unstable to acid and a basic substance, and (3) a sustained-release part containing a compound unstable to acid and a basic substance and having a film that dissolves at pH 6.5 or above in combination.

2. The preparation of claim 1, wherein the film that dissolves at pH 6.5 or above contains one kind or a mixture of two or more kinds selected from the group consisting of hydroxypropyl methylcellulose phthalate, cellulose acetate phthalate, carboxymethylethylcellulose, methyl methacrylate-methacrylic acid copolymer, methacrylic acid-ethyl acrylate copolymer, methacrylic acid-methyl acrylate-methyl methacrylate copolymer, hydroxypropyl methylcellulose acetate succinate, polyvinyl acetate phthalate and shellac.

3. The preparation of claim 1, wherein the sustained-release part comprises a core particle comprising a compound unstable to acid and a basic substance, an intermediate layer formed on the surface of the core particle and a film that dissolves at pH 6.5 or above, which is formed via the intermediate layer.

4. The preparation of claim 1, wherein the compound unstable to acid is a proton pump inhibitor (PPI).

5. The preparation of claim 4, wherein the PPI is a compound represented by the formula (I): 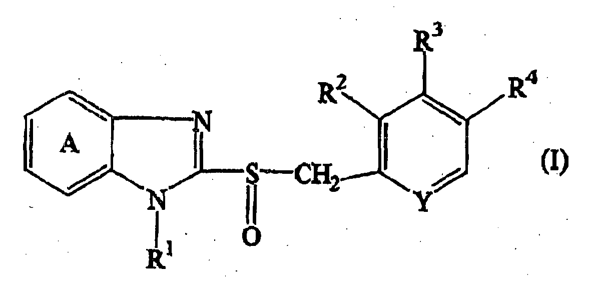 wherein ring A is a benzene ring optionally having substituent(s), R¹ is a hydrogen atom, an aralkyl group optionally having substituent(s), an acyl group or an acyloxy group, R²,R³ and R⁴ are the same or different and each is a hydrogen atom, an alkyl group optionally having substituent(s), an alkoxy group optionally having substituent(s) or an amino group optionally having substituent(s), and Y is a nitrogen atom or CH, or an optically active form thereof or a salt thereof.

6. The preparation of claim 4, wherein the PPI is lansoprazole, omeprazole, rabeprazole, pantoprazole, ilaprazole or an optically active form thereof or a salt thereof.

7. The preparation of claim 1, wherein the antacid is at least one kind of component selected from a metal oxide, a metal hydroxide and an alkaline earth metal carbonate.

8. The preparation of claim 1, wherein a 1% aqueous solution or 1% aqueous suspension of the antacid has a pH of not less than 8.0.

9. The preparation of claim 7, wherein the metal oxide is at least one kind selected from the group consisting of magnesium oxide, magnesium silicate, dry aluminum hydroxide gel and magnesium aluminometasilicate.

10. The preparation of claim 7, wherein the metal hydroxide is at least one kind selected from the group consisting of magnesium hydroxide, aluminum hydroxide, synthetic hydrotalcite, coprecipitate of aluminum hydroxide and magnesium hydroxide, coprecipitate of aluminum hydroxide, magnesium carbonate and calcium carbonate and coprecipitate of aluminum hydroxide and sodium hydrogen carbonate.

11. The preparation of claim 7, wherein the alkaline earth metal carbonate is calcium carbonate or magnesium carbonate.

12. The preparation of claim 1, wherein the content of the antacid is 5 mEq -50 mEq.

13. The preparation of claim 1, wherein the weight ratio of the contents of the compound unstable to acid in the immediate-release part and the sustained-release part is 10:1 - 1:10.

14. The preparation of claim 1, which shows an increase in the intragastric average pH to 4 or above in 0.5 hr after oral administration to a mammal and a retention time at pH 4 or above of not less than 14 hr a day.

15. A solid preparation showing an increase in the intragastric average pH to 4 or above in 0.5 hr after oral administration to a mammal and a retention time at pH 4 or above of not less than 14 hr a day.
